# EUROPEAN PATENT APPLICATION

(11) **EP 4 344 580 A1**
(43) Date of publication of application: **03.04.2024**
(21) Application number: 23199684.4
(22) Date of filing: 26.09.2023
(51) Int. Cl.: A45D 40/16, B65B 3/04, A45D 40/00

(54) **METHOD, MACHINE AND PLANT FOR THE PRODUCTION OF PASTY OR SEMISOLID COSMETIC PRODUCTS WITH A RELIEF SURFACE FINISHING**

(30) Priority: 28.09.2022 IT 202200019965
(71) Applicant: B. Kolormakeup & Skincare S.p.A., 24047 Treviglio (BG) (IT)
(72) Inventor: DE LUIGI, Mario, 20129 Milano (MI) (IT)
(74) Representative: Locatelli, Massimo

(57) **Abstract**

A method for the production of pasty or semisolid cosmetic products in pods with a relief surface finishing provides, for forming said relief surface finishing, to dispose the bottom plate (22) containing the pod (18) on a supporting disc (24) provided with means (35) for retaining the bottom plate in place, the disc (24) being vertically movable in an operating position (36) and a punch (28) adapted to impress said relief surface finishing on the pod being present above the disc (24). An elastic canvas (32) adapted to absorb the oils from the material which makes the cosmetic product during the pressing is advantageously disposed between the pod (18) and the punch (28). The punch (28) is advantageously surrounded by a cylindrical extractor (29) vertically movable with respect to it and intended to push downwards, after the pressing step, the bottom plate (22) with the pod, retained on the disc (24) by the retaining means (35), moving away the pod from the punch (28) and the elastic canvas (32).

## Description

The present invention refers to a process for the production of pasty or semisolid cosmetic products with a relief surface finishing.

The invention further refers to a forming station for forming the relief surface finishing of such cosmetic products and to a plant for the production of such cosmetic products, comprising the above-mentioned forming station for forming the relief surface finishing.

Pasty or semisolid cosmetic products, such as for example eye shadows, foundation, etc., are also defined as "poured products" since they are produced starting from a viscous composition consisting of low-melting, medium-melting, high-melting mixtures and natural raw materials, which is heated up to the melting temperature (generally around 90°C) to be poured in the liquid state in a die intended to provide the cosmetic product with the shape of a pod or tablet and then cooled until reaching a solid or semisolid condition suitable for applying on the face or body of the user.

Usually, the pod of cosmetic product is contained in a metal or plastic-material bottom plate, which is in turn inserted in a container, for example a case or a make-up bag, intended to constitute the final packaging of the product.

In addition to these pasty or semisolid cosmetic products, or "poured products", also cosmetic products consisting of pressed powder obviously exist, also in the form of a pod or tablet contained in a bottom plate, which are characterized by a remarkably harder and more compact structure, and which are prepared by pressing the powders directly in the bottom plate, without needing to heat, pour and cool the composition.

Both these typologies of cosmetic products have of course their own positive features and their own issues and each of them is selected and used according to the preferences and/or requirements of the user.

It is often desirable to provide the cosmetic product thus produced with a relief surface finishing, both for aesthetical reasons and for characterizing it with possible logos or writings, identifying the producer and/or a specific brand.

While for the pressed-powder cosmetic products such relief surface finishing can be easily made by directly pressing the visible surface thereof with a punch having in negative/positive the relief to be reproduced (advantageously during the same pressing operation carried out for compacting the product in the form of a pod or tablet inside the bottom plate), a similar operation cannot be performed on a cosmetic product of the "poured" type for various reasons.

First, the poured product, being viscous, under pressure of the punch would laterally flow outside the bottom plate, with unavoidable loss of material and unrepairable deformation or destruction of the pod. Furthermore, the viscosity of the product would prevent or make remarkably difficult the detaching of the pod from the punch since it would tend to remain 'glued' to the punch itself. In any case, even succeeding in detaching the pod from the punch without breaking it, copious residues of cosmetic product would however remain on the surface of the punch, which would make further uses thereof for pressing the subsequent pods practically impossible.

For these reasons, in the field of cosmetics, the technology for producing relief surface finishings on the visible surface of pods or tablets of pasty or semisolid cosmetic products has always been oriented towards the so-called "back injection", using a mould having an internal cavity on the bottom surface of which the relief decoration to be reproduced on the visible surface of the finished product is present, in negative.

The mould is positioned inverted with respect to the configuration the finished pod will have, i.e., with the impression of the decoration (i.e., the relief, in negative, present inside the cavity of the mould) disposed at the bottom.

The mixture heated up to be brought to the liquid state is poured in the mould from above at the one that, in the finished product, should be the lower (i.e., non-visible) surface of the pod of cosmetic product.

The filling of the mould can also occur through a pierced plate, disposed at the top to close the mould, which will then make the bottom plate for supporting and containing the finished cosmetic product.

Once filled the mould with the poured product, it is cooled to bring the mixture to the pasty or semisolid state that the finished cosmetic product should have.

At this point, the mould is rotated by 180 degrees so as to bring the relief portion of the mould (and the corresponding decorated surface of the pod) to the top and the non-visible surface of the pod (and, possibly, the bottom plate which had made a rear closure of the mould and which should make an element for supporting and containing the pod) to the bottom, then the finished cosmetic product is extracted from the mould. This type of technology is described, for example, in the previous Patent Applications US 2008/0143018 A1 and EP 3 669 700 A1, as well as in Patent US RE45998 E.

The "back injection" technology, however, has various drawbacks, including the general complexity of the plant (which, as seen, needs a dedicated handling station wherein the mould is overturned by 180 degrees), the difficulty of extracting the finished cosmetic product from the mould, which often needs a manual intervention, and further also the fact that the risk of a non-optimal detaching of the pod from the mould, due to the viscosity of the mixture even after cooling, with consequent remainder of material attached to the relief internal surface of the mould, and corresponding imperfection of the visible surface of the extracted pod of cosmetic product, still remains.

Finally, it should be considered that the mould within which the mixture of cosmetic product is poured needs to be completely changed to modify the decoration on the surface of the pod, with consequent operating complications and higher costs related to the need to materially make more moulds.

A general object of the present invention is to overcome the above-mentioned drawbacks providing a process and a machinery for the production of pasty or semisolid cosmetic products with a relief surface finishing, which allows to simplify and accelerate the production, increasing the operation reliability of the various components of the plant, and to reach high qualitative and aesthetical standards in the finished product. Here, relief surface finishing means, in general terms, a three-dimensional surface design, which thus can be either protruding from the visible surface of the pod or recessed (in incuse), or both.

In view of such object, it was thought to make, according to the invention, a method for the production of pasty or semisolid cosmetic products with a relief surface finishing, comprising the steps of:
- heating up to a melting temperature a viscous composition adapted to make the cosmetic product and pour the liquid state composition in a containment bottom plate;
- cooling the composition poured in the bottom plate until reaching a pasty or semisolid condition, obtaining a cosmetic product in the form of a pod with a free upper surface;
- pressing the free upper surface of the pod by a punch having a relief design and forming a pod with a relief surface finishing.

According to the invention, a forming station for forming a relief surface finishing, by pressing, on pasty or semisolid cosmetic products in the form of a pod contained in respective bottom plates, has been further made, comprising an input for feeding the pods of cosmetic product to be subjected to said relief surface finishing, an operating position at operating means adapted to carry out the pressing of the pods of cosmetic product to obtain said relief surface finishing on a free upper surface thereof, an output for moving away the pods of cosmetic product with the relief surface finishing, respective paths for accompanying the pods being provided between the input and the operating position and between the operating position and the output, characterized in that said pressing operating means comprise a lower disc for supporting and vertically moving a bottom plate present in said operating position with a pod of cosmetic product to be pressed, a forming assembly vertically movable above the supporting disc and a ribbon of elastic canvas interposed between the free upper surface of the pod of cosmetic product present on the supporting disc and the forming assembly, said supporting disc being provided with means for retaining the bottom plate in place during the conduct of the operations for making the relief surface finishing on the pod of cosmetic product and said forming assembly comprising in turn a punch having a die in the lower part for making the relief surface finishing on the pod of cosmetic product and a cylindrical extractor coaxially surrounding the punch and vertically movable with respect to it.

According to the invention, a plant for the production of pasty or semisolid cosmetic products with a relief surface finishing has been further made, comprising a pouring station wherein a viscous composition adapted to make the cosmetic product is heated up to a melting temperature and poured in the liquid state in a containment bottom plate, a cooling station wherein the composition poured in the bottom plate is cooled until reaching a pasty or semisolid condition to obtain a cosmetic product in the form of a pod with a free upper surface, a forming station in which the relief surface finishing is made on the free upper surface of the pod. In order to clarify the explanation of the innovative principles of the present invention and its advantages with respect to the known art, a possible exemplary embodiment applying such principles will be described below, with the aid of the attached drawings. In the drawings:
Figure 1 schematically illustrates a plant for the production of pasty or semisolid cosmetic products, with the station for forming the relief surface finishing of such cosmetic products according to the present invention in evidence.
Figure 2 represents a perspective view of the forming station for forming the relief surface finishing, partially sectioned at the means for pressing the cosmetic product.
Figure 3 illustrates a front elevation view, partially sectional, of an enlarged detail of the means for pressing the cosmetic product.
Figures 4 to 10 illustrate a front elevation view, partially sectional corresponding to figure 3 of a sequence of the various operating steps of the means for pressing the cosmetic product, adapted to make the above-mentioned relief surface finishing.
Figure 11 represents in more detail a portion of the carrousel with the seats for housing the bottom plates with the cosmetic product in processing.

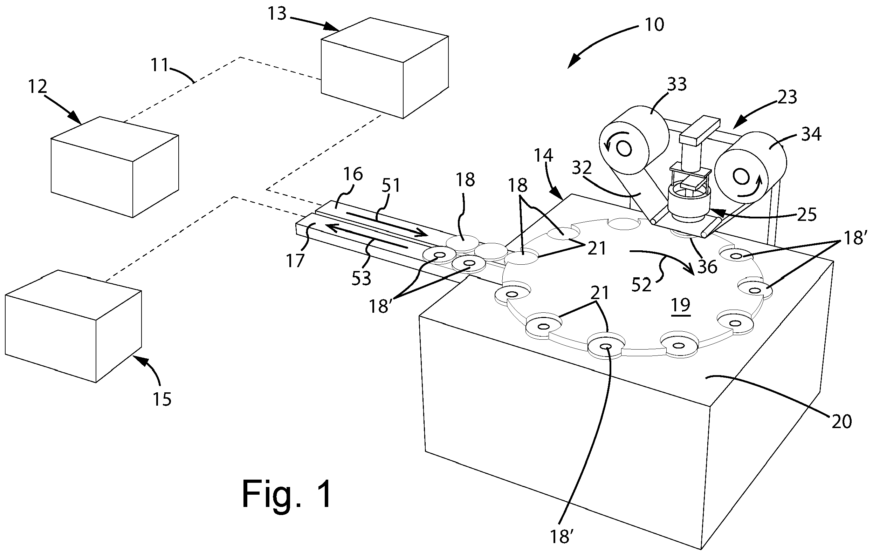

With reference to the figures, figure 1 schematically shows a plant 10 for the production of pasty or semisolid cosmetic products, which sequentially comprises, along an operating path 11, a pouring station 12 wherein the viscous composition intended to make the desired cosmetic product is heated up to its melting temperature to be poured in the liquid state in a die intended to provide the cosmetic product with the shape of a pod or tablet, a cooling station 13 in which the poured cosmetic product is cooled until reaching a solid or semisolid condition suitable for applying on the face or body of the user, a pressing or forming station 14 in which the relief surface finishing is made on the pod of cosmetic product and a discharging station 15 of the finished products.

The stations for pouring and cooling the cosmetic product and the discharging station of the finished products are represented only schematically, without specific details, since they are devices normally used for the production of the poured cosmetic products and therefore their features and operation are well known by those skilled in the art.

Typically, the pouring of the composition intended to make the cosmetic product can occur around 90°C and the cooling of the poured composition at temperatures between -20°C and +25°C, depending on the specific nature and chemical composition of the product. Advantageously, the mixture intended to make the cosmetic product is poured, in the pouring station 12, in dedicated bottom plates adapted to make support and containment for the cosmetic product in pod with a free upper surface, both in the further operating steps in the processing plant and in the subsequent steps of packaging and transporting the finished product.

As shown in figure 1, along the operating path 11 is advantageously present, at an input point of the forming station 14, a charging belt 16 for feeding the pods of cosmetic product to be subjected to the operations for making the relief surface finishing and, at an output point of the forming station 14, a discharging belt 17 for moving away the pods of finished cosmetic product, i.e., processed with the desired relief surface finishing.

For greater clarity and understandability, the pods of cosmetic product being processed in the plant according to the invention will be referred to in the following description, and in the attached drawings, by reference number 18 when referring to the pods not yet subjected to pressing for making the relief surface finishing and by reference number 18' when referring to the pods already subjected to said pressing operation, i.e., the pods of cosmetic product finished and ready to be forwarded to the subsequent steps of moving away from the plant and packaging in the most suitable manners.

Still with reference to figure 1, according to a preferred embodiment, the pressing, or forming, station 14 comprises a carrousel 19 rotatable on a horizontal plane 20 and circumferentially provided with a plurality of substantially semi-circular seats 21, open at the top, at the bottom and radially outwards, adapted to house and accompany along a circular path the pods of cosmetic product 18, 18' contained in the respective bottom plates 22.

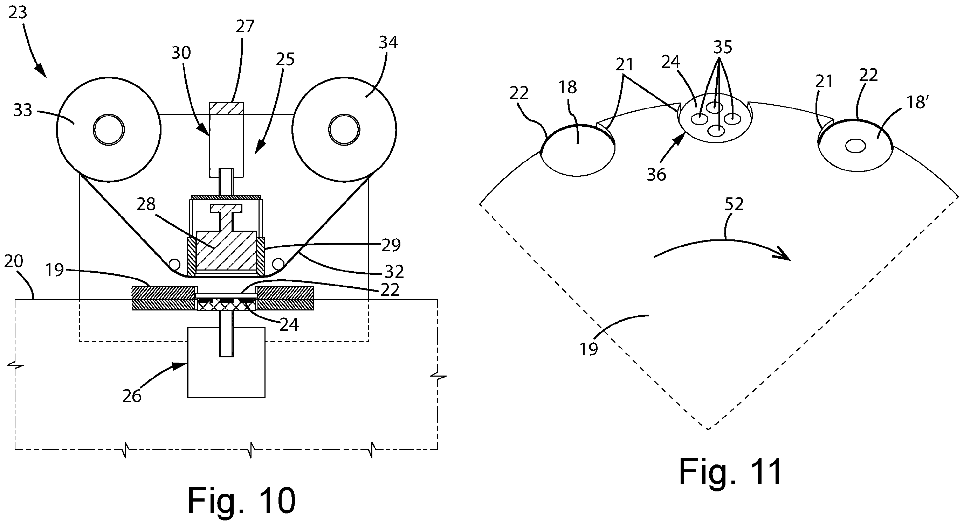

The bottom plates 22 (more visible in the enlarged detail of figure 11) can advantageously be made of tinplate, possibly painted, with thickness between 0.1 mm and 1 mm, diameter between 16 mm and 100 mm and height between 1 mm and 10 mm, depending on the features of the cosmetic product required on the market. The forming station 14 further comprises, along the circular path defined by the above-mentioned seats 21 downstream of the charging belt 16, operating means 23 adapted to carry out the pressing of the pods 18 of poured cosmetic product to obtain the desired relief surface finishing on the free upper face thereof (i.e., the visible face of the cosmetic product contained in the bottom plate 22).

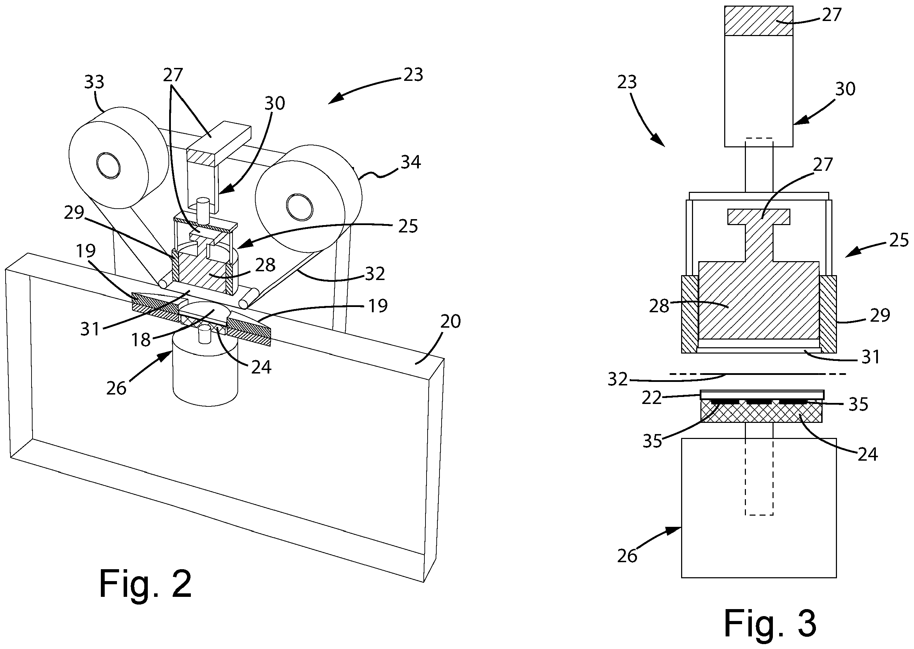

As can be well seen in figure 2 (which shows the forming station sectioned along the vertical plane tangent to the circular path defined by the centres of the seats 21 of the carrousel 19 at the operating position where the pressing of the pods is carried out), the above-mentioned pressing operating means 23 essentially comprise a lower disc 24 for supporting the bottom plate with the pod 18 of cosmetic product to be pressed and a forming assembly 25 disposed above the disc 24. The disc 24 is vertically movable by means of a pneumatic cylindrical actuator 26 placed below the disc itself and the plane 20 for rotating the carrousel 19, while the forming assembly 25 is supported by a vertically movable structure 27, only partially shown in the drawings.

The forming assembly 25 comprises in turn a punch 28, integral with said vertically movable structure 27 and having a die in the lower part with the imprint corresponding to the relief surface finishing to be made on the upper surface of the cosmetic product, and a cylindrical or ring-shaped extractor 29 which coaxially surrounds the punch 28 and whose function will be elucidated in more detail below.

The cylindrical or ring-shaped extractor 29 is vertically movable with respect to the punch 28 by means of a pneumatic cylindrical actuator 30 whose body is integral with the structure 27 for supporting the forming assembly 25 and whose rod is instead integral with the cylindrical extractor which surrounds the punch. Advantageously, the cylindrical extractor 29 is internally shaped, at the lower end thereof, with a 'stepped' circumferential widening (visible in more detail in figure 3) which identifies a seat 31 intended to house the upper edge of the bottom plate 22 containing the pod 18 of cosmetic product during the pressing step, thus ensuring the correct centring of the bottom plate itself with respect to the punch 28.

The pressing operating means 23 further comprise a ribbon of elastic canvas 32 interposed between the free upper surface of the pod 18 of cosmetic product present on the disc 24 and the forming assembly 25. The ribbon of elastic canvas 32 can horizontally advance on command below the forming assembly 25 unwinding from a roller 33 for supplying the blank canvas and, simultaneously, winding on a roller 34 for storing the exhausted canvas.

Features and functions of the ribbon of elastic canvas 32 will be elucidated in more detail below.

According to the invention, the disc 24 for supporting and vertically moving the bottom plate 22 is provided with means 35 for retaining the bottom plate in place during the conduct of the operations for making the relief surface finishing on the pod 18 of cosmetic product.

Advantageously, said retaining means 35 can consist of magnets disposed proximate to the upper surface of the disc 24, for example appearing at the op on it as visible in figure 11, so as to easily and directly interact with the bottom plate 22 made of tinplate. Alternatively, said retaining means 35 can consist of sucker elements, adapted to adhere to the lower surface of the bottom plate 22 even if this was made of other non-ferromagnetic material.

Now, the operation of the plant and, more specifically, of the pressing or forming station 14, will be described, in its essential steps.

The bottom plates 22 containing the pods 18 of poured cosmetic product produced, according to the known technologies, in the pouring and cooling stations 12, 13 reach the pressing or forming station 14 by means of the charging belt 16 moving in the direction of the arrow 51 represented in figure 1 and from here they are charged, one by one, on the carrousel 19, individually housed in the circumferential seats 21 thereof.

The step-by-step rotation of the carrousel 19 in the direction of the arrow 52 represented in figure 1 and figure 11, actuated each time the operating cycle for pressing a pod has been completed, sequentially brings the pods 18 in a position 36 where the above-described pressing operating means 23 are.

At this operating position 36, the horizontal plane 20 on which the bottom plates 22 are dragged by the carrousel in rotation is interrupted and the disc 24 for supporting and vertically moving the bottom plate, provided with the above-mentioned retaining means 35, is present below it. For graphical reasons, in figure 11 the bottom plate which should be in the operating position 36 has been omitted, so as to make the supporting disc 24 and the relative retaining means 35 (advantageously magnets flush to the upper surface of said disc) visible.

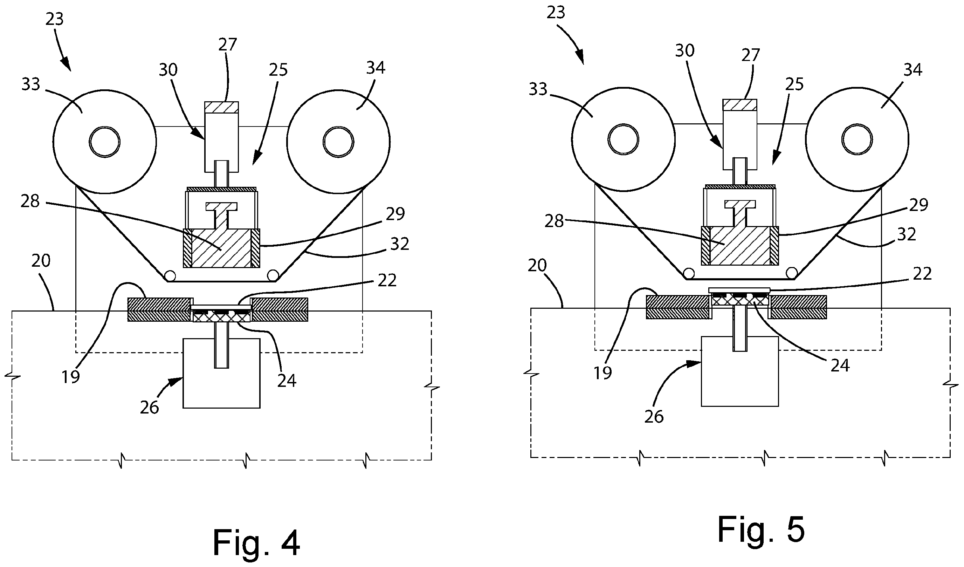

In figure 4 the pressing operating means 23 are represented in the position they assume at the beginning of the pressing operating cycle. This situation will be referred to in the following description also as "zero position".

In particular, the supporting disc 24 is in a lowered position, at the level of the plane 20 on which the circular path for accompanying the pods 18, 18' dragged by the carrousel 19 in rotation is identified. The bottom plate 22 with the pod 18 to be pressed is disposed on the disc 24, in the seat 21 of the carrousel at the operating position 36, kept in place by the retaining means 35.

The forming assembly 25 above the ribbon of elastic canvas 32 is in a raised position, spaced from it.

Figure 5 shows the initial step of the operation of forming the relief surface finishing of the pod of cosmetic product, in which the disc 24, with the bottom plate 22 kept in place by the retaining means 35, is raised (by the respective cylindrical actuator 26) in the direction of the elastic canvas 32.

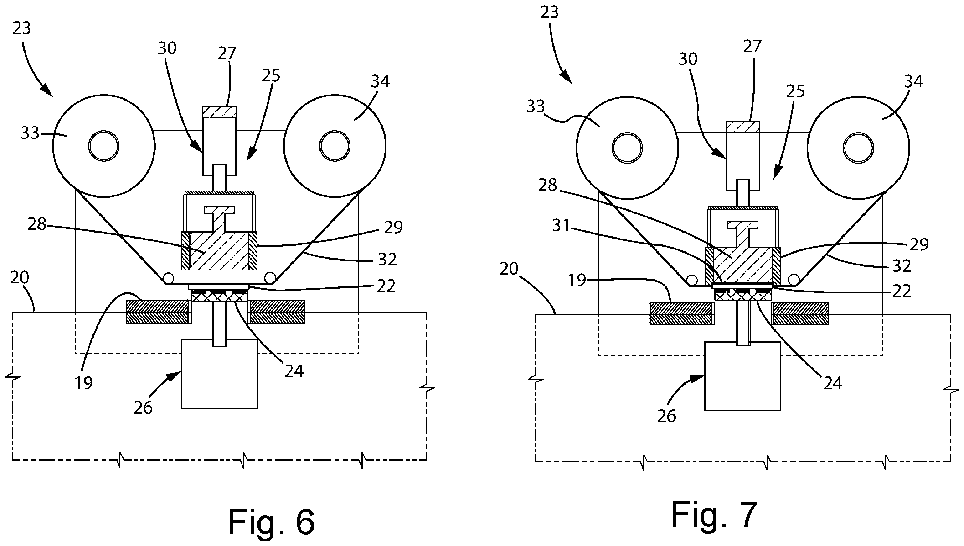

In figure 6 the disc 24 with the bottom plate 22 further rises, until the upper surface of the pod 18 to be pressed reaches the elastic canvas 32.

At this point (figure 7), after the pod 18 is come into contact with the elastic canvas 32 (or even simultaneously to the rising of the disc 24), the entire forming assembly 25 is lowered through the movable structure 27 up to bring the lower edge of the cylindrical extractor 29 also in contact with the elastic canvas 32, and the disc 24 with the bottom plate 22 is then further pushed upwards, causing the bottom plate to be inserted in the centring seat 31 provided at the lower end of the cylindrical extractor 29, with the canvas 32 interposed, suitably deformed due to its elasticity, between the punch 28 and the pod 18 of cosmetic product to be pressed.

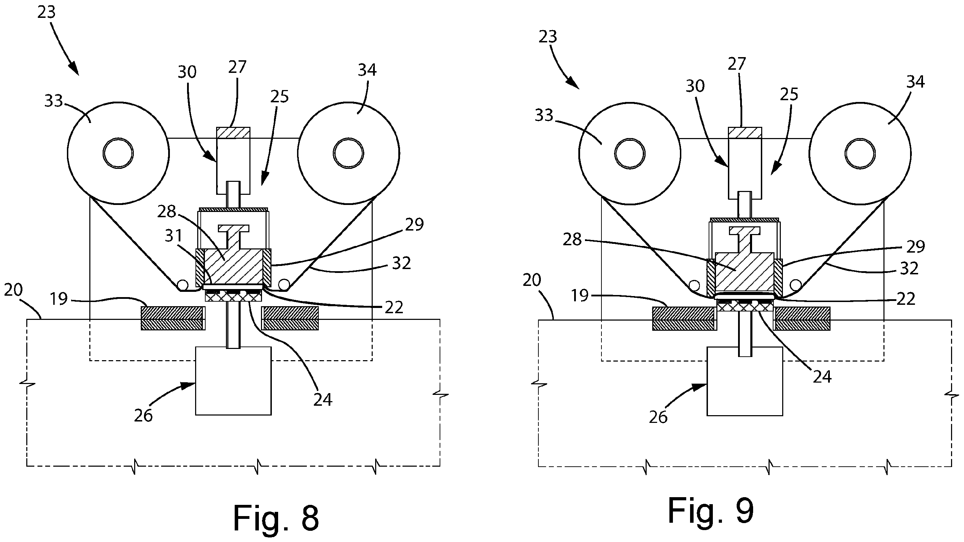

Figure 8 shows the actual pressing step, where the disc 24, with the bottom plate 22 inserted and centred in the above-mentioned lower seat 31 of the cylindrical extractor 29, is further pushed upwards together with the extractor itself, pressing against the punch 28 with the canvas 32 interposed between the punch 28 and the pod 18 of cosmetic product to be pressed. Advantageously, the pressure actuated by the punch on the pod of cosmetic product is between 2 bars and 8 bars.

The desired relief surface finishing, for graphical simplicity represented in a totally schematic and exemplary manner on the free upper surface of the pods 18' in figures 1 and 11, is thus formed on the upper surface of the pod of cosmetic product.

In this step, the elastic canvas 32 plays an active role. Indeed, it helps, due to its texturing, to make the surface finishing of the cosmetic product in pod 18', but especially advantageously allows to absorb and eliminate the oils deriving from pressing of the pod (which, as said, is made starting from mixtures of waxes and silicones, thus viscous and intrinsically oily materials), keeping clean both the visible surface of the pod of cosmetic product (thus ensuring a high aesthetical value thereof) and the surface of the punch 28 which can be thus effectively used for pressing the subsequent pods without needing specific cleaning operations.

At the end of each pressing cycle, indeed, the ribbon of canvas is advanced by a step, unwinding from the supplying roller 33 and winding on the roller 34 for storing the exhausted canvas, so as to always have, for each new pressing operation, a portion of 'blank' canvas between the forming assembly 25 and the bottom plate 22 with the new pod of cosmetic product to be processed.

For example, using a material made of fibres of polyester and elastane, for example 900 of polyester and 10% of elastane, was found to be particularly effective for the elastic canvas 32. Preferably, the elastic canvas 32 has one or more of the following features:
- Mass/area unit: 100-200 g/m² (ISO 3801:1977);
- Permeability to air (speed): 500-600 mm/sec (UNI EN ISO 9237:1997);
- Permeability to air (volumetric flow): 20,000-50,000 l/min/m² (UNI EN ISO 9237:1997).

Advantageously, the thickness of the canvas 32 can be selected between 0.2 mm and 0.5 mm, preferably about 0.3 mm, so that, also due to its elasticity, the canvas itself can perfectly adapt to the interface shape between the bottom plate 22 with the pod of cosmetic product, the matrix consisting of the die of the punch 28 and the lower edge of the cylindrical extractor 29 with its shaped seat 31.

At the end of the pressing step, the cylindrical extractor 29 is lowered with respect to the punch 28 (as visible in figure 9), so as to push downwards the disc 24 with the bottom plate 22 containing the pod of cosmetic product 18' provided with the desired relief surface finishing, thereby detaching the pod itself from the punch and from the elastic canvas 32 (with the aid of the retaining means 35, which keep the bottom plate 22 integral with the disc 24).

In this step of processing the pod, the action of the cylindrical extractor 29, in combination with the means 35 for retaining the bottom plate 22 in place on the disc 24, is also particularly advantageous.

Indeed, since the elastic canvas 32, against its positive role of forming the surface finishing of the pod and absorbing the oils deriving from pressing the material making the cosmetic product, would tend (precisely because of the viscosity of such material) to keep the bottom plate with the processed pod attached to itself, the push of the bottom plate 22 downwards deriving from lowering the cylindrical extractor 29 with respect to the punch 28, in addition to the action of the retaining means 35, allows to make a smooth detaching of the bottom plate with the processed pod 18' from the punch itself, keeping, due to the presence of the elastic canvas 32, the relief decorated surface of the pod perfectly intact.

In practice, the cylindrical extractor 29 assists the retaining means 35 (in a preferred embodiment, magnets cooperating with a bottom plate made of tinplate) in detaching the bottom plate 22 with the processed pod 18' from the lower surface of the punch, so that the bottom plate itself remains integral with the vertically movable disc 24.

Figure 10 shows the return of the disc 24 with the bottom plate 22 containing the processed pod 18' in the lowered position, at the level of the carrousel 19, it had in the "zero position" at the beginning of the cycle.

At this point, the forming assembly 25 can be also brought back in the initial raised position and the ribbon of elastic canvas 32 advanced towards the storing roller 34 by an amount sufficient to dispose a new portion of 'blank' canvas below the punch 28. In this manner, the pressing operating means 23 are brought back in the initial configuration of figure 4, ready to start a new pressing cycle.

Meanwhile, the carrousel 19 is rotated by a step, in the direction of the arrow 52 visible in figure 1 and figure 11, to move away the just processed pod 18', inside its seat 21 in the carrousel, from the operating position 36. The horizontal push on the bottom plate 22 by the carrousel 19 in rotation is indeed sufficient to overcome the attraction vertical force exerted from the disc 24 with the retaining means 35 with respect to the bottom plate itself.

The rotation of the carrousel 19 of course allows a new bottom plate, containing a pod 18 to be processed, to reach the operating position 36 and the furthest one of the already processed pods 18' to be discharged, with their own bottom plate 22, on the discharging belt 17 to be forwarded along the operating path 11 in the direction of the arrow 53 represented in figure 1 and finally reach the discharging station 15 of the finished products.

At this point, it is clear that the present invention allows to reach the prefixed objects. Indeed, due to the presence of the retaining means 35 on the disc 24, assisted by the cylindrical extractor 29 and the elastic canvas 32, the pod of poured cosmetic product contained in the bottom plate 22 can be pressed by the punch 28 and then smoothly detached from it without the risk that the cosmetic product, necessarily provided with a specific viscosity, laterally flows outside the bottom plate damaging the shape and the aesthetical aspect of the pod itself and without the risk that part of the material which makes the cosmetic product remains 'glued' to the punch.

Obviously, the above description of an embodiment applying the innovative principles of the present invention is reported by way of an example of such innovative principles and thereby should not be taken as limiting the protection scope claimed here.

For example, the bottom plates 22 and the pods of cosmetic product contained therein, and thus also the seats 21 of the carrousel 19 and the components of the forming assembly 25 (i.e., the punch 28 and the extractor 29), which are depicted here in a circular shape, could also be made with other shapes, for example squared or polygonal. In such case, obviously, the extractor 29 cannot be strictly defined "cylindrical extractor", but more generally "extractor" or "ring-shaped extractor".

The means 35 for retaining the bottom plate 22 in place on the disc 24, rather than in the form of magnets or suckers, could consist of suitable elements for mechanically clamping the bottom plate by the disc.

Conveyor belts, shuttles, or other linear conveying means could also be used instead of a rotating carrousel 19, and in such case, the feeding of the bottom plates 22 with the pods 18 from the input 16 to the operating position 36 and/or the moving away of the bottom plates 22 with the pods 18' from the operating position 36 to the output 17 could occur along non-circular, but for example rectilinear, accompanying paths. Indeed, this would always fall within the operation principles of the forming station according to the invention, in particular as concerns the action of the pressing operating means 23.

The pouring 12, cooling 13, forming 14 (and possibly also discharging 15) stations could be intended as internal modules of a single machine structure and, in such case, the operating path 11 for the bottom plates containing the pods, schematically represented in figure 1, should be seen as a set of suitable connections between said modules, inside the above-mentioned machine structure.

Finally, the same operating path 11 could also be only theorical, not materially existing, and the passage of the pods from a station to another be manually performed by an operator.

## Claims

1. Method for the production of pasty or semisolid cosmetic products with a relief surface finishing, comprising the steps of:
- heating up to a melting temperature a viscous composition adapted to make the cosmetic product and pour the liquid state composition in a containment bottom plate (22);
- cooling the composition poured in the bottom plate (22) until reaching a pasty or semisolid condition, obtaining a cosmetic product in the form of a pod (18) with a free upper surface;
- pressing the free upper surface of the pod (18) by a punch (28) having a relief design and forming a pod (18') with a relief surface finishing.

2. Method for the production of pasty or semisolid cosmetic products with a relief surface finishing according to claim 1, **characterized in that**, in the pressing step, an elastic canvas (32) is interposed between the free upper surface of the pod (18) of cosmetic product and the punch (28), destined to come into contact, during the pressing, with the cosmetic product and with the punch, absorbing oily substances deriving from pressing of the pod (18) of pasty or semisolid cosmetic product.

3. Method for the production of pasty or semisolid cosmetic products with a relief surface finishing according to claim 2, **characterized in that**, in order to make the pressing of the free upper surface of the pod (18), the following steps are performed:
- feeding the bottom plate (22) containing the pod (18) of cosmetic product to an operating position (36) of a forming station (14) comprising a forming assembly (25) equipped with said punch (28), vertically movable in the operating position (36), and with a cylindrical extractor (29), coaxially surrounding the punch (28) and vertically movable with respect to it, placing the bottom plate (22) on a supporting disc (24) provided with means (35) for retaining the bottom plate in place, the supporting disc (24) being placed, vertically movable, below said forming assembly (25) and said elastic canvas (32) being positioned horizontally between the free upper surface of the pod (18) of cosmetic product present on the supporting disc (24) and the above forming assembly (25);
- raising, starting from an initial lowered position, the supporting disc (24) with the bottom plate (22) containing the pod (18) of cosmetic product up to contact between the free upper surface of the pod (18) and the elastic canvas (32) and lowering, starting from an initial raised position, the forming assembly (25) down to contact of the lower edge of the cylindrical extractor (29) with the elastic canvas (32);
- pushing further upwards the supporting disc (24) with the bottom plate (22), together with the cylindrical extractor (29), with respect to the punch (28), thereby pressing the pod (18) against the punch (28) with the elastic canvas (32) interposed therebetween, impressing the relief surface finishing on the upper surface of the pod (18);
- lowering the cylindrical extractor (29) with respect to the punch (28), pushing downwards the supporting disc (24), which retains by said retaining means (35) the bottom plate (22) containing the pod (18') of cosmetic product with the relief surface finishing, thereby detaching the pod itself from the punch (28) and the elastic canvas (32);
- bringing back the supporting disc (24) with the bottom plate (22) containing the pod (18') of cosmetic product with the relief surface finishing in the initial lowered position and bringing back the forming assembly (25) in the initial raised position;
- moving away the bottom plate (22) containing the pod (18') of cosmetic product with the relief surface finishing from the operating position (36).

4. Method for the production of pasty or semisolid cosmetic products with a relief surface finishing according to claim 3, **characterized in that**, when the bottom plate (22) containing the pod (18) of cosmetic product and the lower edge of the cylindrical extractor (29) are in contact with the elastic canvas (32), before pressing the pod (18) against the punch (28), the supporting disc (24) with the bottom plate (22) is further raised with respect to the forming assembly (25), thereby inserting the bottom plate (22) in a centring seat (31) provided at the lower end of the cylindrical extractor (29), with the canvas (32) interposed between the punch (28) and the pod (18) of cosmetic product.

5. Method for the production of pasty or semisolid cosmetic products with a relief surface finishing according to claim 3, **characterized in that**, after detaching the pod (18') of cosmetic product with the relief surface finishing and the punch (28), the elastic canvas (32) previously interposed therebetween is translated in order to dispose a new portion of blank canvas below the punch (28).

6. Method for the production of pasty or semisolid cosmetic products with a relief surface finishing according to claim 5, **characterized in that** the translation of the elastic canvas (32) is made by unwinding a ribbon of elastic canvas from a roller (33) for supplying blank canvas and simultaneously winding the ribbon on a roller (34) for storing exhausted canvas.

7. Method for the production of pasty or semisolid cosmetic products with a relief surface finishing according to claim 3, **characterized in that** the supplying of the bottom plate (22) containing the pod (18) of cosmetic product to the operating position (36) of the forming station (14) and the moving away of the bottom plate (22) containing the pod (18') of cosmetic product with the relief surface finishing from said operating position (36) are made by dragging of the bottom plates (22) by a rotating carrousel (19), provided with seats (21) for housing the bottom plates, along a circular path passing through said operating position (36).

8. Forming station for forming a relief surface finishing, by pressing, on pasty or semisolid cosmetic products in the form of a pod contained in respective bottom plates (22), comprising an input (16) for feeding the pods (18) of cosmetic product to be subjected to said relief surface finishing, an operating position (36) at operating means (23) adapted to carry out the pressing of the pods (18) of cosmetic product to obtain said relief surface finishing on a free upper surface thereof, an output (17) for moving away the pods (18') of cosmetic product with the relief surface finishing, respective accompanying paths of the pods (18, 18') being provided between the input (16) and the operating position (36) and between the operating position (36) and the output (17), **characterized in that** said pressing operating means (23) comprise a lower disc (24) for supporting and vertically moving a bottom plate (22) present in said operating position (36) with a pod (18) of cosmetic product to be pressed, a forming assembly (25) vertically movable above the supporting disc (24) and a ribbon of elastic canvas (32) interposed between the free upper surface of the pod (18) of cosmetic product present on the supporting disc (24) and the forming assembly (25), said supporting disc (24) being provided with means (35) for retaining the bottom plate (22) in place during the conduct of the operations for making the relief surface finishing on the pod (18) of cosmetic product and said forming assembly (25) comprising in turn a punch (28) having a die in the lower part for making the relief surface finishing on the pod (18) of cosmetic product and a cylindrical extractor (29) coaxially surrounding the punch (28) and vertically movable with respect to it.

9. Forming station for forming a relief surface finishing on pasty or semisolid cosmetic products according to claim 8, **characterized in that** it further comprises a carrousel (19) rotatable on a horizontal plane (20) and circumferentially provided with a plurality of substantially semi-circular seats (21), open at the top, at the bottom and radially outwards, adapted to house and accompany along a circular path between the input (16) and the operating position (36) the bottom plates (22) with the pods (18) of cosmetic product to be subjected to said relief surface finishing and between the operating position (36) and the output (17) the bottom plates (22) with the pods (18') of cosmetic product with the made relief surface finishing.

10. Forming station for forming a relief surface finishing on pasty or semisolid cosmetic products according to claim 8, **characterized in that** the cylindrical extractor (29) is internally shaped, at the lower end thereof, with a stepped circumferential widening which identifies a seat (31) intended to house the upper edge of a bottom plate (22) containing a pod (18) of cosmetic product during the pressing step.

11. Forming station for forming a relief surface finishing on pasty or semisolid cosmetic products according to claim 8, **characterized in that** the ribbon of elastic canvas (32) can be horizontally translated on command to be unwound from a roller (33) for supplying the blank canvas and wound on a roller (34) for storing the exhausted canvas, passing below the forming assembly (25).

12. Forming station for forming a relief surface finishing on pasty or semisolid cosmetic products according to claim 8, **characterized in that** the means (35) for retaining the bottom plate (22) in place on the disc (24) consist of magnets disposed proximate to the upper surface of the disc (24).

13. Forming station for forming a relief surface finishing on pasty or semisolid cosmetic products according to claim 12, **characterized in that** said magnets appear at the top on the upper surface of the disc (24).

14. Forming station for forming a relief surface finishing on pasty or semisolid cosmetic products according to claim 12, **characterized in that** the bottom plates (22) are made of tinplate to magnetically react in attraction with the magnets on the disc (24).

15. Forming station for forming a relief surface finishing on pasty or semisolid cosmetic products according to claim 8, **characterized in that** the means (35) for retaining the bottom plate (22) in place on the disc (24) consist of sucker elements, adapted to adhere to the lower surface of the bottom plate (22).

16. Plant for the production of pasty or semisolid cosmetic products with a relief surface finishing, comprising a pouring station (12) wherein a viscous composition adapted to make the cosmetic product is heated up to a melting temperature and poured in the liquid state in a containment bottom plate (22), a cooling station (13) wherein the composition poured in the bottom plate (22) is cooled until reaching a pasty or semisolid condition to obtain a cosmetic product in the form of a pod (18) with a free upper surface, a forming station (14) according to claims 8-15 wherein the relief surface finishing is made on the free upper surface of the pod (18).

17. Plant for the production of pasty or semisolid cosmetic products with a relief surface finishing according to claim 16, **characterized in that** it further comprises, downstream of the forming station (14), a discharging station (15) of the finished products.

18. Plant for the production of pasty or semisolid cosmetic products with a relief surface finishing according to claim 16 or 17, **characterized in that** the pouring station (12), the cooling station (13), the forming station (14) and the possible discharging station (15) are sequentially connected by an operating path (11) for the bottom plates (22) containing the pods (18, 18').
